# EUROPÄISCHE PATENTANMELDUNG

(11) **EP 1 129 703 A2**
(43) Veröffentlichungstag der Anmeldung: **05.09.2001**
(21) Anmeldenummer: 01102969.1
(22) Anmeldetag: 08.02.2001
(51) Int. Cl.: A61K 7/50

(54) **Wässrige oder wässrig-alkoholische Körperreinigungsmittel enthaltend Sorbitolester**

(30) Priorität: 04.03.2000 DE 10010768
(71) Anmelder: Clariant GmbH, 65929 Frankfurt am Main (DE)
(72) Erfinder: Henning, Torsten, Dr., 65779 Kelkheim (DE); Mulitze-Kleinheyer, Vera, 65926 Frankfurt am Main (DE)

(57) **Zusammenfassung**

Wässrige oder wässrig-alkoholische Körperreinigungsmittel enthaltend Sorbitolester, erhalten durch Umesterung von gegebenenfalls oxalkyliertem Sorbit mit Fettsäuremethylestern oder Fettsäuretriglyceriden und gegebenenfalls Oxalkylierung der durch Umesterung mit Fettsäuremethylestern erhaltenen Reaktionsprodukten. Die Sorbitolester dienen als Solubilisierungsmittel von Wirkstoffen, als Stabilisator, sowie als pflegende und rückfettende Komponenten.

## Beschreibung

Die Erfindung betrifft wässrige oder wässrig-alkoholische Körperreinigungs- und Körperpflegemittel zur milden Reinigung und Pflege der Haut, gekennzeichnet durch einen Gehalt an Sorbitolester, erhalten durch Umesterung von gegebenenfalls oxalkyliertem Sorbit mit Fettsäuremethylestern oder Fettsäuretriglyceriden und gegebenenfalls Oxalkylierung der durch Umesterung mit Fettsäuremethylestern erhaltenen Reaktionsprodukte.

Körperreinigung und -pflege in zwei Schritten ist zeitraubend, so dass von vielen Verbrauchern Mittel mit reinigender und pflegender Wirkung zugleich bevorzugt werden.
Eine Vielzahl von kosmetischen Produkten versucht diesem Anspruch gerecht zu werden.

In US 5 612 307 werden wässrige, flüssige Körperreinigungsmittel beansprucht, die neben üblichen Tensidsystemen eine pflegende Komponente aus der Gruppe der Siliconöle, Fette, Öle, Wachse, hydrophoben Pflanzenextrakte, Fettsäuren, Alkohole, Ester, Lipide und/oder Phospholipide, enthalten.
In WO 94/03152 werden Duschgels beansprucht, bestehend im wesentlichen aus einem Tensid, Siliconöl und einem kationischen Polymer.
Unbefriedigend ist, dass pflegende und feuchtigkeitsspendende Komponenten nicht in ausreichender Menge in wässrige Reinigungsmittelformulierungen eingearbeitet werden können. Ein weiteres Problem ist, dass sich wässrige Dispersionen aus Tensidsystemen und feuchtigkeitsspendenden und pflegenden Komponenten im Laufe der Zeit separieren und somit wenig lagerstabil sind.

In DE 197 27 950 und DE 199 06 368 sind Emulgatoren beschrieben, die erhalten werden durch Umesterung von gegebenenfalls oxalkyliertem Sorbit mit Fettsäuremethylestern oder Fettsäuretriglyceriden oder Oxalkylierung der durch Umesterung mit Fettsäuremethylestern erhaltenen Reaktionsprodukte.

Es bestand daher die Aufgabe, neue hautpflegende Körperreinigungsmittel zu entwickeln, die reinigende und pflegende Wirkung in sich vereinen und die frei sind von den oben genannten Nachteilen.
Gegenstand der Erfindung sind wässrige oder wässrig-alkoholische Körperreinigungsmittel, enthaltend Sorbitolester, erhalten durch Umesterung von gegebenenfalls oxalkyliertem Sorbit mit Fettsäuremethylestern oder Fettsäuretriglyceriden und gegebenenfalls Oxalkylierung der durch Umesterung mit Fettsäuremethylestern erhaltenen Reaktionsprodukte.

Die erfindungsgemäß in wässrigen oder wässrig-alkoholischen kosmetischen Mitteln eingesetzten Sorbitolester werden vorzugsweise erhalten durch Reaktion von Sorbit mit Fettsäuremethylestern oder Fettsäuretriglyceriden entsprechend dem in DE 197 27 950 beschriebenen Verfahren, wobei als Katalysatoren hier allerdings übliche alkalische Katalysatoren, insbesondere Natriummethylat eingesetzt werden. Der Fettsäurerest in den Fettsäuremethylestern und Fettsäuretriglyceriden enthält im allgemeinen 8 bis 22 C-Atome und kann geradkettig oder verzweigt, gesättigt oder ungesättigt sein. Beispiele hierfür sind Palmitinsäure, Stearinsäure, Laurinsäure, Linolsäure, Linolensäure, Isostearinsäure oder Ölsäure. Als Fettsäuretriglyceride kommen alle nativen tierischen oder pflanzlichen Öle, Fette und Wachse in Frage, beispielsweise Olivenöl, Rapsöl, Palmkernöl, Sonnenblumenöl, Kokosöl, Leinöl, Ricinusöl, Sojabohnenöl, gegebenenfalls auch in raffinierter oder hydrierter Form. Da diese natürlichen Fette, Öle und Wachse normalerweise Mischungen von Fettsäuren mit unterschiedlicher Kettenlänge darstellen, gilt dies auch für die Fettsäurereste in den erfindungsgemäß eingesetzten Sorbitolestern.

Die Umsetzung von Sorbit mit den Fettsäuretriglyceriden oder Methylestern geschieht im Eintopfverfahren ohne Lösemittel bei Temperaturen von ungefähr 120 - 140°C in Gegenwart eines alkalischen Katalysators. Die Reaktionszeit beträgt im allgemeinen 12 bis 13 Stunden. Bei der Verwendung von Fettsäuremethylester wird während dieser Reaktion das entstehende Methanol abdestilliert. Da Sorbit üblicherweise als wässrige Lösung im Handel ist, muss zunächst das Wasser entfernt werden. Dies geschieht durch Destillation bei maximal 120°C unter vermindertem Druck. Das Molverhältnis von Sorbit zu Fettsäuremethylester beträgt im allgemeinen 1:1 bis 1:2. Bei Verwendung von Fettsäuretriglyceriden beträgt das Molverhältnis im allgemeinen 1 mol Sorbit auf 1 bis 4,5 mol, vorzugsweise 3,5 bis 4,5 mol, Fettsäuretriglycerid.

Die Umsetzungsprodukte können auch alkoxyliert sein, vorzugsweise ethoxyliert, der Gehalt an Ethoxylatgruppen kann 1 bis 90 -CH₂CH₂O-Gruppen auf ein Molekül Sorbit betragen. Die Einführung der Alkoxylatgruppen kann erfolgen durch eine Alkoxylierung des Sorbits nach an sich bekannten Verfahren vor der Umesterungsreaktion. Bei der Umesterung mit Fettsäuremethylestern arbeitet man jedoch vorzugsweise so, dass man zunächst die Umesterung durchführt und anschließend nach an sich bekannten Verfahren die Alkoxylatgruppen einführt.

Das Reaktionsprodukt dieser Umesterungsreaktion besteht neben Restmengen an nicht umgesetztem Sorbit im wesentlichen aus den Sorbitmonofettsäureestern und den Sorbitdifettsäureestern. Die entsprechenden Triester entstehen nur in untergeordneten Mengen. Bei der Verwendung von Fettsäuretriglyceriden als Ausgangsprodukt enthält das Reaktionsprodukt auch noch Mono- und Di-Fettsäureglycerid sowie nicht umgesetztes Triglycerid in Abhängigkeit von dem jeweils gewählten Molverhältnis der Ausgangsverbindungen.

Die so erhaltene Mischung der verschiedenen Reaktionsprodukte eignet sich sehr gut als Solubilisierungsmittel von Wirkstoffen, sowie als Stabilisator und bewirkt einen hervorragenden Rückfettungseffekt in kosmetischen und pharmazeutischen Mitteln.
Die Menge der erfindungsgemäßen Umesterungsprodukte als Rückfettungs-, Solubilisier- und Stabilisiermittel in wässrigen und wässrig-alkoholischen Mitteln beträgt im allgemeinen 0,1 bis 50 Gew.-%, bevorzugt 0,5 bis 10 Gew.-%, insbesondere 1 bis 4 Gew.-%, bezogen auf das fertige Mittel.

Die erfindungsgemäßen Körperreinigungs- und -pflegemittel liegen nicht in Form von Emulsionen vor, es handelt sich beispielsweise um Shampoos, Duschbäder, Duschgels, Schaumbäder, enthaltend oben genannte Sorbitolester oder Mischungen aus verschiedenen Sorbitolestern. Sie können mit allen üblichen anionischen, kationischen, zwitterionischen, nicht-ionischen und amphoteren Tensiden in wässrigem oder wässrig-alkoholischem Medium kombiniert werden.
Die Gesamtmenge der in den erfindungsgemäßen Mitteln eingesetzten Tenside kann zwischen 5 bis 70 Gew.-%, bevorzugt zwischen 10 und 40 Gew.-%, besonders bevorzugt zwischen 12 und 35 Gew.-%, bezogen auf das fertige Mittel sein.

Als anionische waschaktive Substanzen seien genannt: C₁₀-C₂₀-Alkyl- und Alkylencarboxylate, Alkylethercarboxylate, Fettalkoholsulfate, Fettalkoholethersulfate, Alkylamidsulfate und -sulfonate, Fettsäurealkylamidpolyglykolethersulfate, Alkansulfate, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Amphoacetate- oder -glycinate, Acylglutamate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie analogen Alkylammonium-Salze.

Der Gewichtsanteil der anionischen Tenside in den erfindungsgemäßen Mitteln liegt im Bereich von 0,1 % bis 50 %, bevorzugt 7 bis 30 %, besonders bevorzugt 9 bis 18%.

Geeignete kationische Tenside sind beispielsweise quartäre Ammoniumsalze wie Di-(C₁₀-C₂₄-Alkyl)-dimethyl-ammonium-chlorid oder -bromid, vorzugsweise Di-(C₁₂-C₁₈-Alkyl)-dimethyl-ammonium-chlorid oder -bromid; C₁₀-C₂₄-Alkyl-dimethyl-ethylammonium-chlorid oder -bromid; C₁₀-C₂₄-Alkyl-trimethyl-ammonium-chlorid oder -bromid, vorzugsweise Cetyl-trimethyl-ammonium-chlorid oder -bromid und C₂₀-C₂₂-Alkyl-trimethyl-ammonium-chlorid oder -bromid; C₁₀-C₂₄-Alkyl-dimethylbenzyl-ammonium-chlorid oder -bromid, vorzugsweise C₁₂-C₁₈-Alkyl-dimethylbenzyl-ammonium-chlorid; N-(C₁₀-C₁₈-Alkyl)-pyridinium-chlorid oder -bromid, vorzugsweise N-(C₁₂-C₁₆-Alkyl)-pyridinium-chlorid oder -bromid; N-(C₁₀-C₁₈-Alkyl)-isochinolinium-chlorid, -bromid oder -monoalkylsulfat; N-(C₁₂-C₁₈-Alkylpolyolaminoformylmethyl)-pyridinium-chlorid; N-(C₁₂-C₁₈-Alkyl)-N-methyl-morpholinium-chlorid, -bromid oder -monoalkylsulfat; N-(C₁₂-C₁₈-Alkyl)-N-ethyl-morpholinium-chlorid, -bromid oder -monoalkylsulfat; C₁₆-C₁₈-Alkyl-pentaoxethyl-ammonium-chlorid; Diisobutyl-phenoxyethoxyethyldimethylbenzylammonium-chlorid; Salze des N,N-Diethylaminoethylstearylamids und -oleylamids mit Salzsäure, Essigsäure, Milchsäure, Zitronensäure, Phosphorsäure; N-Acyl-aminoethyl-N,N-diethyl-N-methyl-ammoniumchlorid, -bromid oder -monoalkylsulfat und N-Acylaminoethyl-N,N-diethyl-N-benzyl-ammonium-chlorid, -bromid oder -monoalkylsulfat, wobei Acyl vorzugsweise für Stearyl oder Oleyl steht.

Der Gewichtsanteil der kationischen Tenside in den erfindungsgemäßen Mitteln liegt im Bereich von 1 bis 10 %, bevorzugt 2 bis 7 %, besonders bevorzugt 3 bis 5 %.

Als nichtionische Tenside, die als waschaktive Substanzen eingesetzt werden können, kommen beispielsweise in Betracht: Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (Pluronics®); Fettsäureamidpolyethylenglykole; N-Alkyl-, N-Alkoxypolyhydroxyfettsäureamide, insbesondere Fettsäure-N-methylglucamide, Saccharoseester; Polyglykolether, Alkylpolyglycoside, Phosphorsäureester (Mono-, Di- und Triphosphorsäureester ethoxyliert und nicht-ethoxyliert).

Der Gewichtsanteil der nichtionischen Tenside in den erfindungsgemäßen Mitteln liegt im Bereich von 1 bis 20 %, bevorzugt 2 bis 10 %, besonders bevorzugt 3 bis 7 %.

Bevorzugte Amphotenside sind: N-(C₁₂-C₁₈-Alkyl)-β-aminopropionate und N-(C₁₂-C₁₈-Alkyl)-β-iminodipropionate als Alkali- und Mono-, Di- und Trialkylammonium-Salze; N-Acylaminoalkyl-N,N-dimethyl-acetobetain, vorzugsweise N-(C₈-C₁₈-Acyl)aminopropyl-N,N-dimethylacetobetain; C₁₂-C₁₈-Alkyl-dimethyl-sulfopropyl-betain; Amphotenside auf Basis Imidazolin (Handelsname: Miranol®, Steinapon®), vorzugsweise das Natrium-Salz des 1-(β-Carboxy-methyloxyethyl)-1-(carboxymethyl)-2-lauryl-imidazoliniums; Aminoxide, z.B. C₁₂-C₁₈-Alkyldimethylaminoxid, Fettsäureamidoalkyl-dimethylaminoxid.

Der Gewichtsanteil der amphoteren Tenside in den erfindungsgemäßen Mitteln liegt im Bereich von 0,5 bis 20 %, bevorzugt 1 bis 10 %.

Des weiteren können in den erfindungsgemäßen Mitteln schaumverstärkende Co-Tenside aus der Gruppe Alkylbetaine, Alkylamidobetaine, Aminopropionate, Aminoglycinate, Imidazoliniumbetaine und Sulfobetaine, Aminoxide und Fettsäurealkanolamide oder Polyhydroxyamide eingesetzt werden.

Bevorzugte Tenside in den erfindungsgemäßen Mitteln sind Laurylsulfat, Cocoamidopropylbetain, Natriumcocoylglutamat, Di-natriumlaurethsulfosuccinat und Cocosfettsäurediethanolamid.
Die erfindungsgemäßen Zubereitungen können außerdem weitere in der Kosmetik gebräuchliche Zusätze wie Überfettungsmittel, Stabilisatoren, biogene Wirkstoffe, Glycerin, Konservierungsmittel, Perlglanzmittel, Farb- und Duftstoffe, Lösungsmittel, Trübungsmittel, Verdickungsmittel und Dispergiermittel ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, deodorierende Mittel, Stoffe mit keratolytischer und keratoplastischer Wirkung, Enzyme und Trägersubstanzen enthalten, sowie feuchtigkeitsspendende Stoffe. Des weiteren können den erfindungsgemäßen Mitteln antimikrobiell wirkende Agentien zugesetzt werden.

Als Überfettungsmittel können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Typische Beispiele für Fette sind Glyceride, als Wachse kommen u.a. Bienenwachs, Paraffinwachs oder Mikrowachse, gegebenenfalls in Kombination mit hydrophilen Wachsen, z.B. Cetylstearylalkohol in Frage.

Als Stabilisatoren können Metallsalze von Fettsäuren, wie z.B. Magnesium-, Aluminium- und/oder Zinkstearat eingesetzt werden. Unter biogenen Wirkstoffen sind beispielsweise Pflanzenextrakte und Vitaminkomplexe zu verstehen.
Als Konservierungsmittel eignen sich beispielsweise Phenoxyethanol, Formaldehydlösung, Parabene, Pentandiol oder Sorbinsäure.
Als Farbstoffe können die für kosmetische Zwecke geeigneten und zugelassenen Substanzen verwendet werden.
Als Verdickungsmittel eignen sich Natrium-, Kalium-, Ammoniumchlorid, Natriumsulfat, Fettsäurealkylolamide, Cellulosederivate, beispielsweise Hydroxyethylcellulose, Guar Gum, Polyvinylalkohol, Polyvinylpyrrolidon, Hydroxypropyl guar gum, Stärke und Stärkederivate sowie natürliche Gummen, Carboxyvinylpolymere, beispielsweise Carbopol® 934, -940, -941,-956,-980,-981, -1342, -1382.
Als Verdickungs- und Dispergiermittel besonders geeignet sind Ethylenglykolester von Fettsäuren mit 14 bis 22, besonders bevorzugt 16 bis 22 Kohlenstoffatomen, insbesondere Mono- und Di-ethylenglycolstearat. Bevorzugt sind auch Stearinmonoethanolamid, Stearindiethanolamid, Stearinisopropanolamid, Stearinmonoethanolamidstearat, Stearylstearat, Cetylpalmitat, Glycerylstearat, Stearamiddiethanolamiddistearat, Stearamidmonoethanolamidstearat, N,N-Dihydrocarbyl (C₁₂-C₂₂, insbesondere C₁₆-C₁₈)-amidobenzoesäure und deren lösliche Salze, N,N-di(C₁₆-C₁₈)amidobenzoesäure und Derivate.
Die Dispergiermittel werden in Konzentrationen von 0,5 bis 10 Gew.-%, bevorzugt von 0,5 bis 5 Gew.-%, besonders bevorzugt von 1 bis 4 Gew.-%, bezogen auf das fertige Mittel eingesetzt.

Die gewünschte Viskosität der Mittel kann durch Zugabe von Wasser und/oder organischen Lösungsmitteln oder durch Zugabe einer Kombination aus organischen Lösungsmitteln und Verdickungsmitteln eingestellt werden.
Prinzipiell kommen als organische Lösungsmittel alle ein- oder mehrwertigen Alkohole und ethoxylierten Alkohole in Betracht. Bevorzugt werden Alkohole mit 1 bis 4 Kohlenstoffatomen wie Ethanol, Propanol, Isopropanol, n-Butanol und Iso-Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45 Gew.-% und von Polyethylenglykol mit einer relativen Molekülmasse zwischen 400 und 600 in Mengen von 0,5 bis 15 Gew.-% bevorzugt. Weitere geeignete Lösungsmittel sind beispielsweise Triacetin (Glycerintriacetat) und 1-Methoxy-2-propanol.
Die erfindungsgemäßen Mittel können Alkohole und ethoxylierte Alkohole in den Gewichtsmengen von 0,1 % bis 50 % enthalten.

Als Trägermaterialien in Betracht kommen pflanzliche Öle, natürliche und gehärtete Öle, Wachse, Fette, Wasser, Alkohole, Polyole, Glycerol, Glyceride, flüssige Paraffine, flüssige Fettalkohole, Sterol, Polyethylenglykole, Cellulose und Cellulose-Derivate.

Als fungizide Wirkstoffe können Ketoconazol, Oxiconazol, Bifonazol, Butoconazol, Cloconazol, Clotrimazol, Econazol, Enilconazol, Fenticonazol, Isoconazol, Miconazol, Sulconazol, Tioconazol, Fluconazol, Itraconazol, Terconazol, Naftifin und Terbinafin, Zn-Pyrethion und Octopyrox eingesetzt werden.

Als deodorierende Stoffe können Allantoin und Bisabolol in den Gewichtsmengen 0,0001 % bis 10 % eingesetzt werden.
Um die Affinität des/der erfindungsgemäß eingesetzten Sorbitolester auf der Haut zu verbessern können kationische Guar-Polymere in den Gewichtsmengen 0,01 bis 1,0 %, bevorzugt 0,02 bis 0,4 % eingesetzt werden, wie in WO 97/26854 beschrieben.

Geeignete kationische Polymere sind beispielsweise kationische Cellulosederivate, kationische Stärke, Copolymere von Diallylammoniumsalzen und Acrylamiden, quaternierte VinylpyrrolidonNinylimidazol-Polymere, Kondensationsprodukte von Polyglykolen und Aminen, quaternierte Kollagenpolypeptide, quaternierte Weizenpolypeptide, Polyethylenimin, kationische Siliconpolymere wie z.B. Amidomethicone, Copolymere der Adipinsäure und Dimethylaminohydroxypropyldiethylentriamin, Polyaminopolyamid und kationische Chitinderivate wie beispielsweise Chitosan.

Geeignete Siliconverbindungen sind beispielsweise Dimethylpolysiloxan, Methyl-phenylpolysiloxane, cyclische Silicone sowie amino-, fettsäure-, alkohol-, polyether-, epoxy-, fluor- und/oder alkylmodifizierte Siliconverbindungen, sowie Polyalkylsiloxane, Polyalkylarylsiloxane, Polyethersiloxan-Copolymere, wie in US 5 104 645 und darin zitierten Schriften beschrieben, die bei Raumtemperatur sowohl flüssig als auch harzförmig vorliegen können.

Die erfindungsgemäßen Mittel können mit konventionellen Ceramiden, Pseudoceramiden, Fettsäure-N-alkylpolyhydroxyalkylamiden, Cholesterin, Cholesterinfettsäureestern, Fettsäuren, Triglyceriden, Cerebrosiden, Phospholipiden und ähnlichen Stoffen, abgemischt werden.
Als perlglanzgebende Verbindungen geeignet sind Fettsäuremonoalkanolamide, Fettsäuredialkanolamide, Monoester oder Diester von Alkylenglycol, insbesondere des Ethylenglykols und/oder Propylenglykols oder dessen Oligomere mit höheren Fettsäuren, z.B. Palmitinsäure, Stearinsäure oder Behensäure oder Mischungen davon. Mono- oder Diester von Alkylenglykolen mit Fettsäuren, Fettsäuren und deren Metallsalze, Monoester oder Polyester von Glycerin mit Carbonsäuren und Ketosulfone verschiedener Art. In den erfindungsgemäßen Mitteln sind als perlglanzgebende Komponente besonders bevorzugt Ethylenglycoldistearat und Polyethylenglykoldistearat mit 3 Glykoleinheiten.
Als feuchtigkeitsspendende Substanz stehen beispielsweise Isopropylpalmitat, Glycerin und/oder Sorbitol zu Verfügung, die in den Gewichtsmengen 0,1 bis 50 % eingesetzt werden können.

Die Herstellung der erfindungsgemäßen Mittel erfolgt in an sich bekannter Weise durch Zusammengeben der einzelnen Komponenten und eine - soweit erforderlich - der jeweiligen Zubereitungsart angepasste Weiterverarbeitung. Einige dieser vielfältigen möglichen Zubereitungsformen werden in den Ausführungsbeispielen beispielhaft beschrieben. Mit dem erfindungsgemäßen Einsatz von Sorbitolester in wässrigen oder wässrig-alkoholischen Körperreinigungsmitteln, insbesondere in Duschbädern, Duschgels und Schaumbädern lassen sich Schädigungen der Haut vermeiden und die Hautfreundlichkeit der Mittel verbessern.

Die nachfolgenden Beispiele sollen den Gegenstand der Erfindung näher erläutern, ohne ihn darauf einzuschränken.

### Beispiele

### Beispiel 1: Duschgel

| | Komponente | Gew.-% |
|---|---|---|
| 1 | Carbopol ETD 2020 | 1,5 |
| 2 | Polyquaternium-10 | 0,3 |
| 3 | Glycerin | 2,0 |
| 4 | Emulsogen SRO | 2,0 |
| 5 | Genagen LDA | 9,2 |
| 6 | Genagen CAB | 4,0 |
| 7 | Hostapon CLG | 4,8 |
| 8 | Zitronensäure | 0,5 |
| 9 | Methyldibromoglutaronitril/Phenoxyethanol | 0,05 |
| 10 | Parfüm | 0,5 |
| 11 | Trübungsmittel Opacifyer 641 | 0,8 |
| 12 | E-Wasser | ad 100 |

Komponenten 1 und 2 wurden vorgelegt und in ca. 70°C heißem E-Wasser unter Rühren gelöst. Nacheinander wurden 3, 4, 5, 6 und 7 unter Rühren zugegeben und der pH-Wert mit Zitronensäure auf pH 6,0 eingestellt. Durch Zugabe von 9 und 10 wurde das Mittel konserviert und parfümiert und anschließend mit dem Trübungsmittel 11 versehen.

### Beispiel 2: Duschgel

| | Komponente | Gew.-% |
|---|---|---|
| 1 | Carbopol ETD 2020 | 3,0 |
| 2 | Polyquaternium-10 | 0,3 |
| 3 | Emulsogen SRO | 3,0 |
| 4 | Medialan LD | 2,0 |
| 5 | Genagen LAA | 7,2 |
| 6 | Genagen CAB | 4,0 |
| 7 | Hostapon KCG | 6,9 |
| 8 | Milchsäure | 0,5 |
| 9 | Konservierungsmittel | q.s. |
| 10 | Parfüm | q.s. |
| 11 | Genapol TSM | 1,0 |
| 12 | E-Wasser | ad 100 |

Komponenten 1 und 2 wurden vorgelegt und in ca. 70°C heißem E-Wasser unter Rühren gelöst. Nacheinander wurden 3, 4, 5, 6 und 7 unter Rühren zugegeben und der pH-Wert mit Milchsäure auf pH 6,0 eingestellt. Durch Zugabe von 9 und 10 wurde das Mittel konserviert und parfümiert und anschließend mit dem Seidenglanzmittel 11 versehen.

### Verzeichnis der eingesetzten Produkte

| | | |
|---|---|---|
| ® Carbopol ETD 2020 | (Clariant GmbH) | Polyacrylsäure, vernetzt |
| SRO® Emulsogen | (Clariant GmbH) | Sorbitester auf Basis von Rapsöl |
| ® Genagen LDA | (Clariant GmbH) | Laurylamphodiacetat, Na-Salz |
| ® Genagen LAA | (Clariant GmbH) | Laurylamphoacetat, Na-Salz |
| ® Genagen CAB | (Clariant GmbH) | Cocoamidopropylbetain |
| ® Hostapon CLG | (Clariant GmbH) | Natriumlaurylglutamat |
| ® Hostapon KCG | (Clariant GmbH) | Natriumcocoylglutamat |
| ® Medialan LD | (Clariant GmbH) | Natriumlauroylsarcosinat |
| ® Genapol TSM | (Clariant GmbH) | PEG-3 Distearat, Natriumlaurethsulfat |
| ® Opacifier 641 | | Na-Methacrylat-Styrol Copolymer |

Das Produkt Emulsogen SRO (Sorbitolester des Rapsöls) wurde wie folgt hergestellt:
1 Mol Sorbit in Form eines 70 %igen Sorbitsirups wurden vorgelegt und das Wasser im Wasserstrahlvakuum bei 120°C abdestilliert. Nach Zugabe von 1 Gew.-% (bezogen auf die gesamte Einwaage) Kaliumcarbonat (30 %ig in Wasser bei 80°C) wurde bei vollem Wasserstrahlvakuum das Wasser abdestilliert. Anschließend wurden 4 Mol raffiniertes Rapsöl zugegeben und 8 Stunden bei 140°C gerührt.

## Patentansprüche

1. Wässrige oder wässrig-alkoholische Körperreinigungsmittel enthaltend Sorbitolester, erhalten durch Umesterung von gegebenenfalls oxalkyliertem Sorbit mit Fettsäuremethylestern oder Fettsäuretriglyceriden und gegebenenfalls Oxalkylierung der durch Umesterung mit Fettsäuremethylestern erhaltenen Reaktionsprodukte.

2. Körperreinigungsmittel nach Anspruch 1, **dadurch gekennzeichnet**, dass sie Sorbitolester enthalten, die durch Umsetzung von Sorbit mit Fettsäuretriglyceriden erhalten werden.

3. Körperreinigungsmittel nach Anspruch 1, **dadurch gekennzeichnet**, dass sie Sorbitolester enthalten, die durch Umsetzung von 1 mol Sorbit mit 3,5 bis 4,5 mol Fettsäuretriglycerid erhalten werden.

4. Körperreinigungsmittel nach Anspruch 1, **dadurch gekennzeichnet**, dass sie 0,1 bis 50 Gew.-% an Sorbitolester enthalten.
